# EUROPEAN PATENT SPECIFICATION

(11) **EP 0 980 382 B1**
(45) Date of publication and mention of the grant of the patent: **02.01.2003**
(21) Application number: 98918788.5
(22) Date of filing: 28.04.1998
(51) Int. Cl.: C07J 31/00, A61K 31/565, C07J 17/00, C07J 1/00

(54) **ESTROGENIC 19-NORANDROST-17-ONE DERIVATIVES WITH AN AROMATIC B-RING**
ÖSTROGENE 19-NORANDROST-17-ON-DERIVATE MIT EINEM AROMATISCHEN B-RING
DERIVES OESTROGENIQUES 19-NORANDROST-17-ONE RENFERMANT UN CYCLE B AROMATIQUE

(30) Priority: 02.05.1997 US 850427
(43) Date of publication of application: 23.02.2000
(73) Proprietor: Wyeth, Madison, New Jersey 07940-0874 (US)
(72) Inventor: SHAH, Syed, Muzafar, East Hanover, NJ 07936 (US); RAVEENDRANATH, Panolil, Monroe, NY 10950 (US); KAGAN, Michael, Z., Plainsboro, NJ 08536 (US)
(74) Representative: Talbott, Dawn Jacqueline
(86) International application number: US9808455
(87) International publication number: WO98050412

(56) References cited:
- DE-C- 635 781
- GB-A- 864 231
- R. JOHNSON ET AL: "Steady State Urinary Excretion Method for Determining Bioequivalence of Conjugated Estrogen Products" JOURNAL OF PHARMACEUTICAL SCIENCES., vol. 67, no. 9, September 1978, WASHINGTON US, pages 1218-1224, XP002070687
- K. JUNGHANS ET AL: "Lösungsmitteleffekte bei der elektrolytischen Reduktion von Steroiden mit aromatischen A- und B- Ring" CHEMISCHE BERICHTE., vol. 112, no. 7, 1979, WEINHEIM DE, pages 2631-2639, XP002070688
- CHEMICAL ABSTRACTS, vol. 106, no. 25, 22 June 1987 Columbus, Ohio, US; abstract no. 207882, M. NUMAZAWA ET AL: "Equilin and equilenin biosynthesis. Stereochemistry of aromatisation of 3-hydroxy-3,5,7-androstatrien-17-one by horse placenta" page 83; column 1; XP002070694 & J. STEROID BIOCHEM., vol. 26, no. 1, 1987, pages 137-143,
- CHEMICAL ABSTRACTS, vol. 66, no. 21, 22 May 1967 Columbus, Ohio, US; abstract no. 92028, L. STARKA ET AL: "Biosynthesis of equilin and equilenin from 7.alpha.-hydroxydehydroepiandrosterone and its sulfate by human placenta maintained in organ culture" page 8600; column 1; XP002070695 & EUR. J. STEROIDES , vol. 1, no. 1, 1966, pages 37-45,
- P. KOCOVSKY ET AL: "The Synthesis of Androstane Derivatives with an Aromatic B-RIng" COLLECTION OF CZECHOSLOVAK CHEMICAL COMMUNICATIONS., vol. 39, no. 7, 1974, PRAGUE CS, pages 1905-1913, XP002070689
- W. E. BACHMANN ET AL: "195. Über die Spaltung der Doppelbindung im Neoergosterol" HELVETICA CHIMICA ACTA., vol. 42, no. 6, 15 October 1959, BASEL CH, pages 1790-1793, XP002070690
- A. H. EIMASRY ET AL: "19-Norsteroids of Unnatural Configuration from Erogesterol" JOURNAL OF PHARMACEUTICAL SCIENCES., vol. 59, no. 4, April 1970, WASHINGTON US, pages 449-458, XP002070691
- W. H. SCHULLER ET AL: "Conversion of Ergosterol into the Estrogen Neoergosterol by Direct Peroxide Cleavage" JOURNAL OF MEDICINAL CHEMISTRY., vol. 14, no. 5, May 1971, WASHINGTON US, page 466 XP002070692
- R. H. PETERS ET AL: "17-Desoxy Estrogen Analogues" JOURNAL OF MEDICINAL CHEMISTRY., vol. 32, no. 7, July 1989, WASHINGTON US, pages 1642-1652, XP002070693

## Description

The use of naturally occurring estrogenic compositions of substantial purity and low toxicity such as PREMARIN™ (conjugated equine estrogens) has become a preferred medical treatment for alleviating the symptoms of menopausal syndrome, osteoporosis/osteopenia in estrogen deficient women and in other hormone related disorders. The estrogenic components of the naturally occurring estrogenic compositions have been generally identified as sulfate esters of estrone, equilin, equilenin, 17-β-estradiol, dihydroequilenin and 17-β-dihydroequilenin (U.S. Patent 2,834,712). The estrogenic compositions are usually buffered or stabilized with alkali metal salts of organic or inorganic acids at a substantially neutral pH of about 6.5 to 7.5. Urea has also been used as a stabilizer (U.S. 3,608,077). The incorporation of antioxidants to stabilize synthetic conjugated estrogens and the failure of pH control with tris(hydroxymethyl)aminomethane (TRIS) to prevent hydrolysis is discussed in U.S. 4,154,820.

One of the compounds described herein, 3β-hydroxy-5,7,9-estratriene-17-one 3-sulfate ester sodium salt is a minor component of PREMARIN™ (conjugated equine estrogens). The preparation of 3β-hydroxy-5,7,9-estratriene-17-one is been disclosed by D. Banerjee in Ind. Chim. Beige. Suppl. 2: 435 (1959); however, no utility is provided for this compound.

### DESCRIPTION OF THE INVENTION

In accordance with this invention, there is provided a pharmaceutically acceptable salt of 3β-hydroxy-5,7,9-estratriene-17-one 3-sulfate ester. The structure of 3β-hydroxy-5,7,9-estratriene-17-one is shown below as compound **I**.

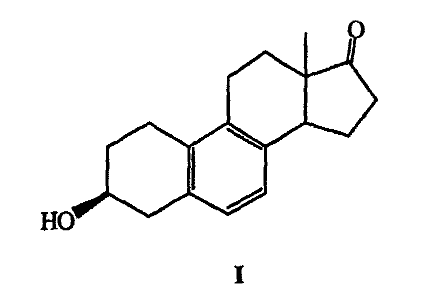

Pharmaceutically acceptable salts of 3β-hydroxy-5,7,9-estratriene-17-one 3-sulfate ester include, but are not limited to, the alkali metal salts, alkaline earth metal salts, ammonium salts, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

Alkali metal salts include sodium and potassium salts, particularly preferred are sodium salts. Alkaline earth metal salts include calcium and magnesium salts. Suitable alkyl groups include methyl, ethyl, propyl, butyl, pentyl and hexyl, preferred alkyl groups being methyl and ethyl. Where more than one alkyl group is present the groups may be the same or different. Preferred trialkylammonium salts are trimethylammonium salts and triethylammonium salts.

There is also provided an alkali metal salt of 3β-hydroxy-5,7,9-estratriene-17-one.

As used in accordance with this invention, treating covers treatment of an existing condition, ameliorating the condition, or providing palliation of the condition and inhibiting includes inhibiting or preventing the progress or development of the condition.

The present invention further provides compositions comprising 3β-hydroxy-5,7,9-estratriene-17-one, 3β-hydroxy-5,7,9-estratriene-17-one 3-sulfate ester or pharmaceutically acceptable salts or a mixture of these. In particular it provides compositions comprising at least 1% of 3β-hydroxy-5,7,9-estratriene-17-one, 3β-hydroxy-5,7,9-estratriene-17-one 3-sulfate ester or pharmaceutically acceptable salts or a mixture of these. One aspect of the present invention provides compositions wherein the only estrogenic agent is 3β-hydroxy-5,7,9-estratriene-17-one, 3β-hydroxy-5,7,9-estratriene-17-one 3-sulfate ester or pharmaceutically acceptable salts or a mixture of these. Embodiments of the present invention include compositions wherein the only active compound is 3β-hydroxy-5,7,9-estratriene-17-one, 3β-hydroxy-5,7,9-estratriene-17-one 3-sulfate ester or pharmaceutically acceptable salts or a mixture of these. In these embodiments other excipients and carriers may be included but no further active materials are included.

The present invention also provides processes for the preparation of 3β-hydroxy-5,7,9-estratriene-17-one, 3β-hydroxy-5,7,9-estratriene-17-one 3-sulfate ester and pharmaceutically acceptable salts of these.

3β-hydroxy-5,7,9-estratriene-17-one or a pharmaceutically acceptable salt thereof may be prepared by the oxidative aromatisation of estra-5(10),7-dien-3β-ol-17-one or estra-5(10),7-dien-3α-ol-17-one using a suitable oxidising agent, such as DEAD (diethyldiazodicarboxylate), DDQ or Pd/C. Alternatively equilenin (or a partially hydrogenated derivative thereof) may be reduced e.g. by metal alcohol reduction (e.g. by the Birch reaction), by metal ammonium reduction or by electrolytic reduction.

3β-hydroxy-5,7,9-estratriene-17-one 3-sulfate ester may be prepared by sulphonating 3β-hydroxy-5,7,9-estratriene-17-one using, e.g. sulphuric acid, chlorosulphuric acid or an amidosulfonic acid.

Alternatively the pH of a salt of 3β-hydroxy-5,7,9-estratriene-17-one 3-sulfate ester may be adjusted to provide the free acid.

A pharmaceutically acceptable salt of 3β-hydroxy-5,7,9-estratriene-17-one or its 3-sulfate ester may be prepared by:
a) converting 3β-hydroxy-5,7,9-estratriene-17-one or its 3-sulfate ester to a pharmaceutically acceptable salt or
b) converting a pharmaceutically acceptable salt of 3β-hydroxy-5,7,9-estratriene-17-one or its 3-sulfate ester to a different pharmaceutically acceptable salt of 3β-hydroxy-5,7,9-estratriene-17-one or its 3-sulfate ester.

3β-hydroxy-5,7,9-estratriene-17-one 3-sulfate ester may be converted to a pharmaceutically acceptable salt by neutralising the acid with an appropriate base, e.g. with an alkali metal carbonate, an alkaline earth metal carbonate or a primary, secondary, tertiary or quaternary amine carbonate. Alkali metal or alkaline earth metal salts may be prepared by using the appropriate alkali metal hydride e.g. sodium hydride, potassium hydride or lithium hydride.

A pharmaceutically acceptable salt of 3β-hydroxy-5,7,9-estratriene-17-one or its 3-sulfate ester may be converted to a different pharmaceutically acceptable salt by displacement, by using an ion exchange resin or by double decomposition (metastasis). Displacement of a weak base with a stronger one may be utilised to convert, e.g. an amine salt to an alkali metal salt or an alkaline earth metal salt using an appropriate base, e.g. a hydroxide. For example a trialkylamine salt such as a triethylamine salt may be converted to an alkali metal salt such as a sodium salt by treating it with an alkali metal hydroxide such as aqueous sodium hydroxide. The displacement may be carried out using an ion exchange resin. Alternatively one salt may be converted to another by double decomposition, e.g. an alkaline earth metal salt such as the calcium salt may be replaced with an alkali metal salt. E.g. the calcium salt of 3β-hydroxy-5,7,9-estratriene-17-one or its 3-sulfate ester may be dissolved in water followed by the addition of e.g. sodium carbonate. Insoluble calcium carbonate would then precipitate out to provide the sodium salt of 3β-hydroxy-5,7,9-estratriene-17-one or its 3-sulfate ester.

A pharmaceutically acceptable salt of 3β-hydroxy-5,7,9-estratriene-17-one 3-sulfate ester may be prepared by directly converting 3β-hydroxy-5,7,9-estratriene-17-one to a pharmaceutically acceptable salt of 3β-hydroxy-5,7,9-estratriene-17-one 3-sulfate ester. This may be performed by reacting it with the appropriate aminesulfurtrioxide complex, e.g. by reacting it with a trialkylaminesulfurtrioxide (such as triethylaminesulfurtrioxide complex) to provide the corresponding trialkylamine salt (such as the triethylamine salt). If desired the salt may then be converted to another salt of the invention as described above. Alkali metal salts may be prepared by treating 3β-hydroxy-5,7,9-estratriene-17-one with the appropriate alkali metal hydride e.g. sodium hydride, potassium hydride or lithium hydride to produce the corresponding alkoxide *in situ* and then adding a trialkylaminesulfurtrioxide (such as triethylaminesulfurtrioxide complex) to provide the corresponding trialkylamine salt (such as the triethylamine salt). If desired the salt may then be converted to another salt of the invention as described above.

The present invention also provides pharmaceutically acceptable salts of 3β-hydroxy-5,7,9-estratriene-17-one 3-sulfate ester prepared by a chemical process, particularly those prepared according to the processes described above. The invention also provides pharmaceutically acceptable salts of 3β-hydroxy-5,7,9-estratriene-17-one 3-sulfate ester obtainable by such processes.

The compounds of this invention can be prepared from readily available starting materials. For example, the preparation of 3β-hydroxy-5,7,9-estratriene-17-one 3-sulfate ester sodium salt can be prepared from 3β-hydroxy-5,7,9-estratriene-17-one according to Scheme I. 3β-Hydroxy-5,7,9-estratriene-17-one can be prepared according to D. Banerjee in Ind. Chim. Belge. Suppl. 2:435 (1959).

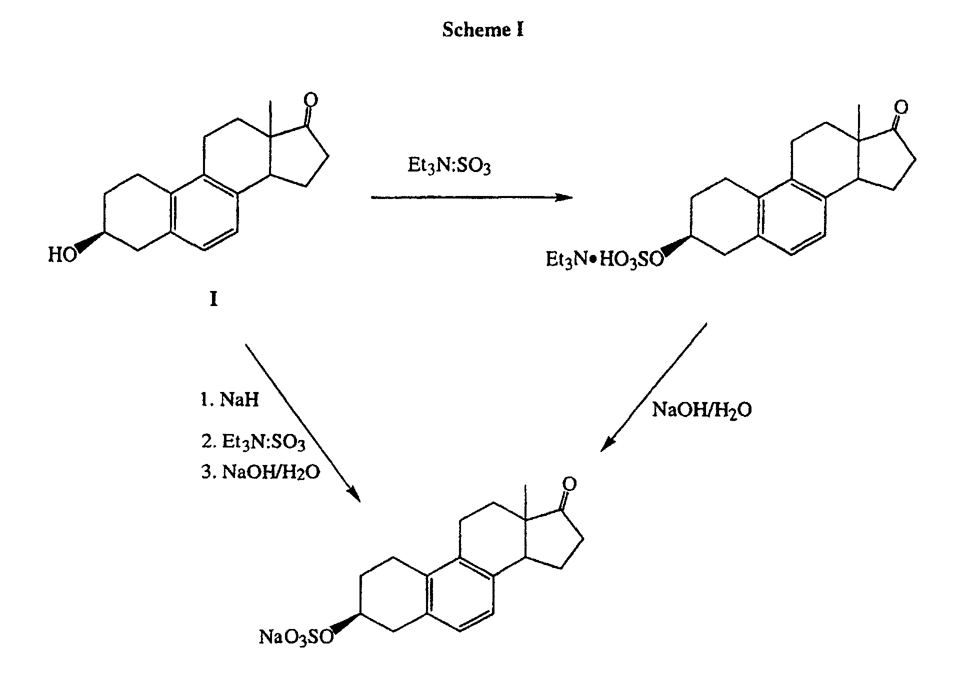

The compounds of this invention are estrogenic, as shown in the in vitro and in vivo standard pharmacological test procedures described below in which 3β-hydroxy-5,7,9-estratriene-17-one was evaluated as a representative compound of this invention.

### Estrogen Receptor Binding

An initial evaluation examined the competitive binding properties of 3β-hydroxy-5,7,9-estratriene-17-one to the human estrogen receptor (hER-α) prepared as a soluble cell extract (cytosol). In this standard pharmacological test procedure, 3β-hydroxy-5,7,9-estratriene-17-one demonstrated no specific binding activity. However, when estrogen receptor binding was analyzed using a whole cell test procedure, specific binding was clearly demonstrated. This test procedure indicated an IC₅₀ of 7.9 x 10⁻⁹ M for 3β-hydroxy-5,7,9-estratriene-17-one. This would be compared with a Kᵢ for estrone, equilin and equilinen of 51, 67 and 375nM, respectively.

### In Vitro Cotransfection Test Procedure

In this standard pharmacological test procedure, hER-α over-expressed in Chinese hamster ovary (CHO) cells infected with adeno-2x-ERE-tk-luciferase, an estrogen responsive reporter gene construct, cells were exposed to varying concentrations (10⁻¹²-10⁻⁵M) of 3β-hydroxy-5,7,9-estratriene-17-one for 24 hours. Cells were also exposed to 17β-estradiol at 10⁻⁹ M. Following the 24-hour treatment, cells were lysed and cell extracts assayed for luciferase activity. The results provided that 3β-hydroxy-5,7,9-estratriene-17-one had an EC₅₀ of approximately 50 nM. Using a similar test procedure, previous data indicate a 5.6nM EC₅₀ for estrone.

### In Vivo Uterotropic Activity

Immature rats were treated with 100 µg 3β-hydroxy-5,7,9-estratriene-17-one for three days (S.C.) as well as additional groups (n=6) of rats treated with 0.5 µg ethinyl estradiol and vehicle as positive and negative controls, respectively. Rats treated with 3β-hydroxy-5,7,9-estratriene-17-one had a uterine weight of 54.63 ± 5.2 mg, whereas control rats had uterine weight of 29.78 ± 1.84 mg demonstrating that 3β-hydroxy-5,7,9-estratriene-17-one was estrogenic. Rats treated with ethinyl estradiol (0.5 µg) had a uterine weight of 92.68 ± 7.6 mg.

The results of these standard pharmacological test procedures demonstrate that the compounds of this invention are estrogenic.

A representative compound of this invention (3β-hydroxy-5,7,9-estratriene-17-one) was evaluated in a standard pharmacological test procedure which measured the cardioprotective effects of the compound tested.

Briefly, Female rats, weighing 180-200 g, were pretreated with DES (0.25 mg/kg, i.p.) 18 hr prior to the study. Uterine horns and aortae were removed from pentobarbital-overdosed animals, cleaned of adherences and cut into 1 cm and 2-3 mm segments, respectively. The preparations were mounted in organ baths containing Jalon solution at 30° C and Krebs solution at 37° C, respectively, and aerated with 95%O₂ and 5% CO₂. The contractions were monitored with force-displacement transducers on a Grass polygraph recorder. DMSO produced no effects on the tissues in the present study and thus, was used as a vehicle for dissolving the test compounds.

Both uterine and aortic smooth muscles were contracted with 60 mM KCl. When potassium-induced contraction reached steady-state, cumulative-concentration response curves were determined for test drug or vehicle. Increasing concentrations of the compound to be evaluated were added when the effect of the previous concentration reached steady state (not longer than 20 min). The EC₅₀ was defined as the concentration of test compound which produced 50% relaxation.

EC₅₀ values of 3.9 x 10⁻⁵ and 4.0 x 10⁻⁵ were obtained in uterine muscle and thoracic aorta, respectively, for 3β-hydroxy-5,7,9-estratriene-17-one. These results demonstrate that the compounds of this invention provide cardioprotective protection, particularly in the treatment of ischemic disease and hypertension.

Based on the results obtained in the standard pharmacological test procedures, the compounds of this invention are useful in providing estrogen replacement therapy following ovariectomy or menopause, and in relieving symptoms related to estrogen deficiency, including vasomotor symptoms, such as hot flushes, and other menopausal related conditions, such as vaginal atrophy, vaginitis, and atrophic changes of the lower urinary tract which may cause increased urinary frequency, incontinence, and dysuria. The compounds of this invention are useful in preventing bone loss and in the inhibition or treatment of osteoporosis. The compounds of this invention are cardioprotective and they are useful in the treatment of atherosclerosis, ischemic disease, and hypertension. These cardiovascular protective properties are of great importance when treating postmenopausal patients with estrogens to prevent osteoporosis and in the male when estrogen therapy is indicated. The compounds of this invention are also antioxidants, and are therefore useful in treating or inhibiting free radical induced disease states. Specific situations in which antioxidant therapy is indicated to be warranted are with cancers, central nervous system disorders, dementias, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke, or injury during reperfusion procedures. Additionally, the compounds of this invention are useful in the suppression of lactation, and in the prophylaxis and treatment of mumps orchitis.

The compounds of this invention can be used alone as a sole therapeutic agent or can be used in combination with other agents, such as other estrogens, progestins, or and androgens.

The compounds of this invention can be formulated neat or with a pharmaceutical carrier for administration, the proportion of which is determined by the solubility and chemical nature of the compound, chosen route of administration and standard pharmacological practice. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally or vaginally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipenniable membrane covering a reservoir containing the active ingredient with or without a carrier, or a matrix containing the active ingredient. Other occlusive devices are known in the literature.

The dosage requirements vary with the particular compositions employed, the route of administration, the severity of the symptoms presented and the particular subject being treated. Based on the results obtained in the standard pharmacological test procedures, projected daily dosages of active compound would be 0.02 µg/kg - 750 µg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The following provides the preparation of 3β-hydroxy-5,7,9-estratriene-17-one 3-sulfate ester sodium salt as a representative compound of this invention.

### EXAMPLE 1

### 3β-Hydroxy-5,7,9-estratriene-17-one 3-sulfate ester sodium salt

### Method A:

To a slurry of sodium hydride (60% dispersion in oil, 0.011 g) in THF (6 mL) was added, under nitrogen, at room temperature, 3β-hydroxy-5,7,9-estratriene-17-one (0.06 g, 0.2 mmol). After stirring for 5 min, triethylamine-sulfurtrioxide complex (0.04 g, 0.21 mmol) was added and stirring continued for 18 h. Solvent was evaporated off and the residue obtained partitioned between water and diethyl ether (6 mL each). The aqueous solution was separated and lyophilized to provide 0.06 g of the desired product.

### Method B:

To a solution of 3β-hydroxy-5,7,9-estratriene-17-one (0.58 g, 2.1 mmol) in THF (15 mL) at RT, under nitrogen, was added triethylamine-sulfur trioxide complex (0.4 g, 2.3 mmol) and stirred for 48 h. The precipitate formed was filtered off and washed with THF (3X3 mL) and dried to provide 3β-hydroxy-5(10),6, 8-estrtriene-17-one-3-triethylammonium sulfate (0.71 g, 73.9%).

To a solution of the triethylammonium salt (0.68 g, 1.5 mmol) was added aqueous sodium hydroxide (1n, 1.6 mL). The solution was stirred for 1h at RT and repeatedly washed with ether (4X10 mL). The aqueous portion was separated and lyophilized to provide 0.56 g of the desired product.
IR (KBr)
3450,2930,1734,1634,1238,1189,1117,1069, 980, 978, 810,620 cm⁻¹
¹H NMR (DMSO-d₆)
6.88 (s, 2H, aromatic), 4.74 (m, 1H, 3-H), 0.64 (s, 3H, 18-Me)
¹³C NMR (DMSO-d₆)
219.14, 134.80, 133.74, 133.43, 132.25, 126.77, 122.48, 70.45, 46.28, 45.77, 36.02, 35.85, 28.72, 28.39, 23.57, 23.45, 21.28, 12.89

## Claims

1. A compound which is a pharmaceutically acceptable salt of 3β-hydroxy-5,7,9-estratriene-17-one 3-sulfate ester.

2. The compound of claim 1 wherein the pharmaceutically acceptable salt of the 3-sulfate ester is an alkali metal salt, alkaline earth metal salt, ammonium salt, alkylammonium salt containing 1-6 carbon atoms, dialkylammonium salt containing 1-6 carbon atoms in each alkyl group, trialkylammonium salt containing 1-6 carbon atoms in each alkyl group or tetraalkylammonium salt containing 1-6 carbon atoms in each alkyl group.

3. A compound according to Claim 1 which is 3β-Hydroxy-5,7,9-estratriene-17-one 3-sulfate ester sodium salt.

4. A pharmaceutically acceptable salt of 3β-hydroxy-5,7,9-estratriene-17-one 3-sulfate ester for use as a pharmaceutical.

5. The compound of claim 4 wherein the pharmaceutically acceptable salt of the 3-sulfate ester is an alkali metal salt, alkaline earth metal salt, ammonium salt, alkylammonium salt containing 1-6 carbon atoms, or dialkylammonium salt containing 1-6 carbon atoms in each alkyl group, trialkylammonium salt containing 1-6 carbon atoms in each alkyl group or tetraalkylammonium salt containing 1-6 carbon atoms in each alkyl group.

6. 3β-Hydroxy-5,7,9-estratriene-17-one for use as a pharmaceutical.

7. An alkali metal salt of 3β-hydroxy-5,7,9-estratriene-17-one.

8. A compound according to Claim 7 which is the sodium salt.

9. A pharmaceutical composition which comprises 3β-hydroxy-5,7,9-estratriene-17-one, 3β-hydroxy-5,7,9-estratriene-17-one 3-sulfate ester or a pharmaceutically acceptable salt thereof and a pharmaceutical carrier.

10. A pharmaceutical composition according to Claim 9 which comprises 3β-hydroxy-5,7,9-estratriene-17-one 3-sulfate ester sodium salt and a pharmaceutical carrier.

11. A pharmaceutical composition according to Claim 9 or Claim 10 comprising at least 1% of a compound as defined therein.

12. A compound as defined in Claim 3 for use as a medicament.

13. Use of a compound as defined in any one of claims 1 to 3 in the preparation of a medicament for:
a) inhibiting or treating free radical induced disease states,
b) inhibiting endogenous free radical involvement in disease development of cancers, central nervous system disorders, dementias, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke, or injury during reperfusion procedures,
c) providing estrogen replacement therapy or treating estrogen deficiency,
d) treating vasomotor symptoms related to estrogen deficiency,
e) treating or inhibiting osteoporosis or
f) treating or inhibiting atherosclerosis in a mammal.

14. Use of 3β-hydroxy-5,7,9-estratriene-17-one in the preparation of a medicament for:
a) inhibiting or treating free radical induced disease states,
b) inhibiting endogenous free radical involvement in disease development of cancers, central nervous system disorders, dementias, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke, or injury during reperfusion procedures,
c) providing estrogen replacement therapy or treating estrogen deficiency,
d) treating vasomotor symptoms related to estrogen deficiency,
e) treating or inhibiting osteoporosis or
f) treating or inhibiting atherosclerosis in a mammal.

15. A process for the preparation of 3β-hydroxy-5,7,9-estratriene-17-one 3-sulfate ester which comprises:
a) sulphonating 3β-hydroxy-5,7,9-estratriene-17-one or
b) adjusting the pH of a salt of 3β-hydroxy-5,7,9-estratriene-17-one 3-sulfate ester to provide the free acid.

16. A process for the preparation of a pharmaceutically acceptable salt of 3β-hydroxy-5,7,9-estratriene-17-one 3-sulfate ester which comprises:
a) converting 3β-hydroxy-5,7,9-estratriene-17-one 3-sulfate ester to a pharmaceutically acceptable salt,
b) converting a pharmaceutically acceptable salt of 3β-hydroxy-5,7,9-estratriene-17-one 3-sulfate ester to a different pharmaceutically acceptable salt of 3β-hydroxy-5,7,9-estratriene-17-one 3-sulfate ester or
c) converting an alkali metal salt of 3β-hydroxy-5,7,9-estratriene-17-one to a pharmaceutically acceptable salt of 3β-hydroxy-5,7,9-estratriene-17-one 3-sulfate ester.

## Patentansprüche

1. Verbindung, welche ein pharmazeutisch annehmbares Salz von 3β-Hydroxy-5,7,9-estratrien-17-on 3-Sulfatester ist.

2. Verbindung nach Anspruch 1, worin das pharmazeutisch annehmbare Salz des 3-Sulfatesters ein Alkalimetallsalz, Erdalkalimetallsalz, Ammoniumsalz, Alkylammoniumsalz, welches 1-6 Kohlenstoffatome enthält, Dialkylammoniumsalz, welches 1-6 Kohlenstoffatome in jeder Alkylgruppe enthält, Trialkylammoniumsalz, welches 1-6 Kohlenstoffatome in jeder Alkylgruppe enthält, oder Tetraalkylammoniumsalz, welches 1-6 Kohlenstoffatome in jeder Alkylgruppe enthält, ist.

3. Verbindung gemäß Anspruch 1, welche 3β-Hydroxy-5,7,9-estratrien-17-on 3-Sulfatester-natriumsalz ist.

4. Pharmazeutisch annehmbares Salz von 3β-Hydroxy-5,7,9-estratrien-17-on 3-Sulfatester zur Verwendung als Pharmazeutikum.

5. Verbindung nach Anspruch 4, worin das pharmazeutisch annehmbare Salz des 3-Sulfatesters ein Alkalimetallsalz, Erdalkalimetallsalz, Ammoniumsalz, Alkylammoniumsalz, welches 1-6 Kohlenstoffatome enthält, oder Dialkylammoniumsalz, welches 1-6 Kohlenstoffatome in jeder Alkylgruppe enthält, Trialkylammoniumsalz, welches 1-6 Kohlenstoffatome in jeder Alkylgruppe enthält, oder Tetraalkylammoniumsalz, welches 1-6 Kohlenstoffatome in jeder Alkylgruppe enthält, ist.

6. 3β-Hydroxy-5,7,9-estratrien-17-on zur Verwendung als Pharmazeutikum.

7. Alkalimetallsalz von 3β-Hydroxy-5,7,9-estratrien-17-on.

8. Verbindung gemäß Anspruch 7, welche das Natriumsalz ist.

9. Pharmazeutische Zusammensetzung, welche 3β-Hydroxy-5,7,9-estratrien-17-on, 3β-Hydroxy-5,7,9-estratrien-17-on 3-Sulfatester oder ein pharmazeutisch annehmbares Salz davon und einen pharmazeutischen Träger umfasst.

10. Pharmazeutische Zusammensetzung gemäß Anspruch 9, welche 3β-Hydroxy-5,7,9-estratrien-17-on 3-Sulfatester-natriumsalz und einen pharmazeutischen Träger umfasst.

11. Pharmazeutische Zusammensetzung gemäß Anspruch 9 oder Anspruch 10, welche mindestens 1% einer Verbindung, wie darin definiert, umfasst.

12. Verbindung, wie in Anspruch 3 definiert, zur Verwendung als Medikament.

13. Verwendung einer Verbindung, wie in einem der Ansprüche 1 bis 3 definiert, bei der Herstellung eines Medikaments zum:
a) Hemmen oder Behandeln von durch freie Radikale verursachten Krankheitszuständen
b) Hemmen endogener Einbeziehung von freien Radikalen in die Krankheitsentwicklung von Krebsen, Störungen des zentralen Nervensystems, Demenzen, Alzheimererkrankung, Knochenerkrankung, Alterung, Entzündungsstörungen, peripher-vaskulärer Krankheit, Rheumatoidarthritis, Autoimmunerkrankung, Atemnot, Emphysem, Verhinderung von Reperfusionsschaden, viraler Hepatitis, chronisch aktiver Hepatitis, Tuberkulose, Psoriasis, systemischem Lupus erythematodes, Atemnotsyndrom des Erwachsenen, Trauma und Schlaganfall des zentralen Nervensystems oder Schäden während Reperfusionsverfahren,
c) Vorsehen von Östrogen-Ersatztherapie oder Behandeln von Östrogenmangel,
d) Behandeln von vasomotorischen Symptomen in Verbindung mit Östrogenmangel,
e) Behandeln oder Hemmen von Osteoporose oder
f) Behandeln oder Hemmen von Atherosklerose
in einem Säuger.

14. Verwendung von 3β-Hydroxy-5,7,9-estratrien-17-on bei der Herstellung eines Medikaments zum:
a) Hemmen oder Behandeln von durch freie Radikale verursachten Krankheitszuständen
b) Hemmen endogener Einbeziehung von freien Radikalen in die Krankheitsentwicklung von Krebsen, Störungen des zentralen Nervensystems, Demenzen, Alzheimererkrankung, Knochenerkrankung, Alterung, Entzündungsstörungen, peripher-vaskulärer Krankheit, Rheumatoidarthritis, Autoimmunerkrankung, Atemnot, Emphysem, Verhinderung von Reperfusionsschaden, viraler Hepatitis, chronisch aktiver Hepatitis, Tuberkulose, Psoriasis, systemischem Lupus erythematodes, Atemnotsyndrom des Erwachsenen, Trauma und Schlaganfall des zentralen Nervensystems oder Schäden während Reperfusionsverfahren,
c) Vorsehen von Östrogen-Ersatztherapie oder Behandeln von Östrogenmangel,
d) Behandeln von vasomotorischen Symptomen in Verbindung mit Östrogenmangel,
e) Behandeln oder Hemmen von Osteoporose oder
f) Behandeln oder Hemmen von Atherosklerose
in einem Säuger.

15. Verfahren für die Herstellung von 3β-Hydroxy-5,7,9-estratrien-17-on 3-Sulfatester, welches umfasst:
a) Sulfonieren von 3β-Hydroxy-5,7,9-estratrien-17-on oder
b) Anpassen des pH eines Salzes von 3β-Hydroxy-5,7,9-estratrien-17-on 3-Sulfatestet, um die freie Säure vorzusehen.

16. Verfahren für die Herstellung eines pharmazeutisch annehmbaren Salzes von 3β-Hydroxy-5,7,9-estratrien-17-on 3-Sulfatester, welches umfasst:
a) Umwandeln von 3β-Hydroxy-5,7,9-estratrien-17-on 3-Sulfatester in ein pharmazeutisch annehmbares Salz,
b) Umwandeln eines pharmazeutisch annehmbaren Salzes von 3β-Hydroxy-5,7,9-estratrien-17-on 3-Sulfatester in ein anderes pharmazeutisch annehmbares Salz von 3β-Hydroxy-5,7,9-estratrien-17-on 3-Sulfatester oder
c) Umwandeln eines Alkalimetallsalzes von 3β-Hydroxy-5,7,9-estratrien-17-on in ein pharmazeutisch annehmbares Salz von 3β-Hydroxy-5,7,9-estratrien-17-on 3-Sulfatester.

## Revendications

1. Composé qui est un sel pharmaceutiquement acceptable d'ester 3-sulfate de 3β-hydroxy-5,7,9-oestradiène-17-one.

2. Composé suivant la revendication 1, dans lequel le sel pharmaceutiquement acceptable d'ester 3-sulfate est un sel de métal alcalin, un sel de métal alcalino-terreux, un sel d'ammonium, un sel d'alkylammonium contenant 1-6 atomes de carbone, un sel de dialkylammonium contenant 1-6 atomes de carbone dans chaque groupement alkyle, un sel de trialkylammonium contenant 1-6 atomes de carbone dans chaque groupement alkyle ou un sel de tétraalkylammonium contenant 1-6 atomes de carbone dans chaque groupement alkyle.

3. Composé suivant la revendication 1, qui est le sel de sodium d'ester 3-sulfate de 3β-hydroxy-5,7,9-oestradiène-17-one.

4. Sel pharmaceutiquement acceptable d'ester 3-sulfate de 3β-hydroxy-5,7,9-oestradiène-17-one pour une utilisation comme agent pharmaceutique.

5. Composé suivant la revendication 4, dans lequel le sel pharmaceutiquement acceptable d'ester 3-sulfate est un sel de métal alcalin, un sel de métal alcalino-terreux, un sel d'ammonium, un sel d'alkylammonium contenant 1-6 atomes de carbone, un sel de dialkylammonium contenant 1-6 atomes de carbone dans chaque groupement alkyle, un sel de trialkylammonium contenant 1-6 atomes de carbone dans chaque groupement alkyle ou un sel de tétraalkylammonium contenant 1-6 atomes de carbone dans chaque groupement alkyle.

6. 3β-hydroxy-5,7,9-oestradiène-17-one pour une utilisation comme agent pharmaceutique.

7. Sel de métal alcalin de 3β-hydroxy-5,7,9-oestradiène-17-one.

8. Composé suivant la revendication 7, qui est le sel de sodium.

9. Composition pharmaceutique qui comprend de la 3β-hydroxy-5,7,9-oestradiène-17-one, de l'ester 3-sulfate de 3β-hydroxy-5,7,9-oestradiène-17-one ou un sel pharmaceutiquement acceptable de ceux-ci et un vecteur pharmaceutique.

10. Composition pharmaceutique suivant la revendication 9, qui comprend le sel de sodium d'ester 3-sulfate de 3β-hydroxy-5,7,9-oestradiène-17-one et un vecteur pharmaceutique.

11. Composition pharmaceutique suivant la revendication 9 ou la revendication 10, comprenant au moins 1% d'un composé comme défini dans ces revendications.

12. Composé comme défini à la revendication 3, pour une utilisation comme médicament.

13. Utilisation d'un composé comme défini dans l'une quelconque des revendications 1 à 3 dans la préparation d'un médicament pour :
a) inhiber ou traiter des états de maladie induits par les radicaux libres,
b) inhiber l'implication des radicaux libres endogènes dans le développement pathologique de cancers, de troubles du système nerveux central, de démences, de la maladie d'Alzheimer, d'une affection osseuse, du vieillissement, de troubles inflammatoires, d'une affection vasculaire périphérique, d'une arthrite rhumatoïde, de maladies auto-immunes, d'une détresse respiratoire, d'un emphysème, d'une prévention de lésion de reperfusion, d'une hépatite virale, d'une hépatite chronique active, de la tuberculose, du psoriasis, du lupus érythémateux disséminé, d'un syndrome de détresse respiratoire de l'adulte, d'un traumatisme au système nerveux central et d'un ictus, ou d'une lésion pendant des procédures de reperfusion,
c) procurer une thérapie de substitution aux oestrogènes ou un traitement d'une carence en oestrogènes,
d) traiter les symptômes vasomoteurs associés à une déficience en oestrogènes,
e) traiter ou inhiber l'ostéoporose ou
f) traiter ou inhiber l'athérosclérose chez un mammifère.

14. Utilisation de 3β-hydroxy-5,7,9-oestradiène-17-one dans la préparation d'un médicament pour :
a) inhiber ou traiter des états de maladie induits par les radicaux libres,
b) inhiber l'implication des radicaux libres endogènes dans le développement pathologique de cancers, de troubles du système nerveux central, de démences, de la maladie d'Alzheimer, d'une affection osseuse, du vieillissement, de troubles inflammatoires, d'une affection vasculaire périphérique, d'une arthrite rhumatoïde, de maladies auto-immunes, d'une détresse respiratoire, d'un emphysème, d'une prévention de lésion de reperfusion, d'une hépatite virale, d'une hépatite chronique active, de la tuberculose, du psoriasis, du lupus érythémateux disséminé, d'un syndrome de détresse respiratoire de l'adulte, d'un traumatisme au système nerveux central et d'un ictus, ou d'une lésion pendant des procédures de reperfusion,
c) procurer une thérapie de substitution aux oestrogènes ou un traitement d'une carence en oestrogènes,
d) traiter les symptômes vasomoteurs associés à une déficience en oestrogènes,
e) traiter ou inhiber l'ostéoporose ou
f) traiter ou inhiber l'athérosclérose chez un mammifère.

15. Procédé pour la préparation d'un ester 3-sulfate de 3β-hydroxy-5,7,9-oestradiène-17-one qui comprend :
a) la sulfonation de la 3β-hydroxy-5,7,9-oestradiène-17-one ou
b) l'ajustement du pH d'un sel d'ester 3-sulfate de 3β-hydroxy-5,7,9-oestradiène-17-one pour obtenir l'acide libre.

16. Procédé pour la préparation d'un sel pharmaceutiquement acceptable d'ester 3-sulfate de 3β-hydroxy-5,7,9-oestradiène-17-one qui comprend ;
a) la conversion de l'ester 3-sulfate de 3β-hydroxy-5,7,9-oestradiène-17-one en un sel pharmaceutiquement acceptable,
b) la conversion d'un sel pharmaceutiquement acceptable d'ester 3-sulfate de 3β-hydroxy-5,7,9-oestradiène-17-one en un sel pharmaceutiquement acceptable différent d'ester 3-sulfate de 3β-hydroxy-5,7,9-oestradiène-17-one ou
c) la conversion d'un sel de métal alcalin de 3β-hydroxy-5,7,9-oestradiène-17-one en un sel pharmaceutiquement acceptable d'ester 3-sulfate de 3β-hydroxy-5,7,9-oestradiène-17-one.
